# EUROPEAN PATENT SPECIFICATION

(11) **EP 3 191 147 B1**
(45) Date of publication and mention of the grant of the patent: **31.10.2018**
(21) Application number: 15762613.6
(22) Date of filing: 10.09.2015
(51) Int. Cl.: A61L 27/30, A61N 1/18

(54) **IMPLANTABLE DEVICE WITH SELECTIVE CELL ADHESION AND METHOD OF PRODUCTION**
IMPLANTIERBARE VORRICHTUNG MIT SELEKTIVER ZELLADHÄSION UND VERFAHREN ZUR HERSTELLUNG
DISPOSITIF IMPLANTABLE À ADHÉRENCE CELLULAIRE SÉLECTIVE ET PROCÉDÉ DE PRODUCTION

(30) Priority: 10.09.2014 LU 92539
(43) Date of publication of application: 19.07.2017
(73) Proprietor: Luxembourg Institute of Science and Technology (LIST), 4362 Esch-sur-Alzette (LU); Luxembourg Institute of Health, 1445 Strassen (LU)
(72) Inventor: LENOBLE, Damien, 69200 Wellin (BE); THOMANN, Jean-Sébastien, 54730 Gorcy (FR); PALISSOT, Valérie, 57100 Thionville (FR)
(74) Representative: Lecomte & Partners
(86) International application number: PCT/EP2015/070754
(87) International publication number: WO 2016/038158

(56) References cited:
- WO-A1-2009/065171
- US-A1- 2011 282 421
- KIM IG-HYEON ET AL: "Formation of V-shaped pits in InGaN/GaN multiquantum wells and bulk InGaN films", APPLIED PHYSICS LETTERS, AMERICAN INSTITUTE OF PHYSICS, US, vol. 73, no. 12, 21 September 1998 (1998-09-21), pages 1634-1636, XP012020972, ISSN: 0003-6951, DOI: 10.1063/1.122229

## Description

### Technical field

The present invention relates to the field of implantable materials and devices. In particular it relates to an implantable device having a substrate on which cell growth and adhesion is selectively enhanced.

### Background of the invention

Several materials and devices have been investigated and proposed to probe or/and restore activities of different biological tissues and cells. The goal of such research is to provide a material or a device able to reconnect for example human neurons with post synaptic cells so that lost neuronal functions may be repaired or improved by implanted devices. As an example, the first successful neuroprosthetics-cochlear implant put to practice in the early 1990s is a neural interface device that uses an electrode array to stimulate the auditory nerve in the cochlea with electric impulses. Electrical stimulation of peripheral nerves has gained a lot of interest from clinicians for the treatment of pain, for restoration of motricity functions and for the treatment of epilepsy. Additionally, the neuroengineering research community has investigated various neural interface devices for the Central Nervous System, CNS. Such devices are designed to regulate mood disorders, epilepsy or symptoms arising with Parkinson's disease, through appropriate electric charge deliveries with deep brain stimulation. Recording of brain signals has been used to control a brain-computer interface, BCI [1]. Nerve autografts [2] are the current clinical standard for peripheral nerve regeneration across large nerve gaps, i.e. gaps having a length longer than 20 mm. Synthetic nerve guidance channels are currently not known for lack of suitable materials that allow for signal transmission and guided growth of cells.

Implantable devices and neural interfaces in particular face several challenges:
- reduce the inflammatory response and gliosis around implants, as these reduce the signal to noise ratio delivered to/from the devices or lead to chronic recording failures [3];
- improve the spatial resolution of neural interfaces that allows the collection and the delivery of signals to or from a portion of the neuron-dense brain;
- selectively stimulate precise areas to induce specific responses from the CNS.

In what follows and throughout the description of the present application, the term biocompatibility will be used to refer to the ability of an implanted device and its component materials to perform their desired function in their implanted environment, maintaining beneficial cellular and tissue responses, and not eliciting undesirable local or systemic effects in the body.

Further, bio-fouling of an implanted device or material refers to the uncontrolled growth of tissue on the device or material.

While several materials have been tested for in vivo bio implant applications, polymers are nowadays the materials of choice due to their plasticity and biocompatibility. In brain-mechanical interfaces, BMI, polymers are mostly used as encapsulation matrices for semi-conducting materials. Several polymers, such as poly(3,4-ethylenedioxythiophene), PEDOT, polyaniline, or polythiophene are known to have conductive properties. The use of conductive polymers can increase the biocompatibility of an implanted material and enable interaction with surrounding cells. However, conductive polymers remain very sensitive to changes in impedance caused by bio-fouling or the uptake of the materials in resident immune cells of the brain [4].

Even though plasticity as found in polymers is important, a minimum of rigidity is nevertheless required to design substrates with the ability to guide and to probe cells, such as neurons.

In applications relating to brain machine interfaces, there is a need for interfaces in which the synaptic connections of neurons can be grown on artificial substrates. The growth should be controlled and precisely directed to defined locations. It has been investigated to use conventional nanotechnology processing steps to create channels, wells or pillars [5] on the surface of semiconductor chips, which led to control cell growth following a designed pattern. Such techniques are particularly suitable for custom networks of neuronal cells, with crosslinks over transducer sites on the surface of electronic chips. Standard Si-based chips have been widely investigated for this purpose, but the lack of chemical stability limits their use in vivo. Therefore, the use of Si-based devices requires specific packaging technology to avoid the rapid silicon oxidation under basic or acidic conditions. Several enzymes are known to react with silicon or its oxide. Indeed, the hydrolysis of SiO₂ usually leads to the release of silicic acid, Si(OH)₄, the toxicity of which is yet unknown at low concentrations. At high concentrations, Si(OH)₄ is suspected to interact with aluminum containing compounds and to contribute to neuronal degeneration. Si-based bio-sensors have been shown to induce inflammation when used in brain implants. Turning to semi-conducting materials, Silicon carbide, SiC, is known to have excellent chemical stability and interesting bio-compatibility properties [6]. Depending on its crystalline phase, the band-gap of SiC varies from 2.3 to 3.2 eV. However, its electronic properties are difficult to fine tune due to the low lattice-defect tolerance. Smaller cross-sectional areas of Si probes have shown a slower astroglial response than larger electrodes. However, the gliotic inflammatory response was similar for all designs after several weeks [7]. Due to its remarkable chemical stability, Gallium nitride, GaN, has been identified as a good candidate for the development of implantable or bio-recording devices [8]. GaN can be functionalized to enable peptide coupling. Specific peptides can be used to promote bio-adhesion or to decrease the risk of tissue inflammation [9].
Some tertiary III-V compounds open the way to design new semiconducting material with tunable opto-electronic properties. Unfortunately, few of them are described in the art as being biocompatible. AIGaN was described as biocompatible in [10], however the ratio of Al/Ga was small, thereby limiting the application windows for this material.
Kim et al. ("Formation of V-shaped pits in InGaN/GaN multiquantum wells and bulk InGaN films", Applied Physics Letters, Volum 73, Number 12, 1998, 1634-1636) disclose describe forming InGaN layers without discussing their bioadhesion properties.
Patent document WO 2009/0654171 A1 discloses an electrode array for a cochlear implant and the use of silicone as biocompatible material.
Patent document US 2011/0282421 A1 discloses devices for neuronal growth wherein a layer of nanodiamond particles is used as a neuronal growth surface.

### Technical problem to be solved

It is therefore an objective of the present invention to provide an implantable device which selectively enhances or promotes cell adhesion thereto while allowing to control the cell growth, and which overcomes at least some of the disadvantages of the prior art.

### Summary of the invention

Any references in the description to methods of treatment refer to the compounds, pharmaceutical compositions and medicaments of the present invention for use in a method for treatment of the human (or animal) body by therapy or for diagnosis.
According to a first aspect of the invention, an implantable device is provided. The device comprises substrate has a surface. A first portion of the surface is used for directly contacting biological cells or tissue. The surface portion comprises In₍ₓ₎Ga₍₁₋ₓ₎N, where x is in the range from 0.001 to 0.999. The first surface portion promotes cell adhesion to the substrate and directs cell growth on the substrate, and further defines a semiconducting component of the device.

The substrate may have several surfaces comprising a portion of the first type.
Preferably, x may be in the range from 0.001 to 0.88 or 0.01 to 0.88.
The first portion may preferably integrally cover the surface of the substrate. The first portion may be contiguous or non-contiguous on said surface.
The surface of the substrate may further preferably comprise a second portion which is distinct from said first portion, wherein said second portion inhibits cell-adhesion to the substrate, and wherein said second portion comprises Indium nitride, InN.
The first and second portions may preferably integrally cover said surface of said substrate. Preferably, the substrate may comprise Indium nitride, InN.
The first and/or second portion may preferably be provided as a thin layer on the surface of the substrate.
Further, the first and/or second portion may preferably be provided as a thick layer on the surface of the substrate.
Even more preferably, the first portion may define a pattern on the surface of the substrate, along which cell adhesion is enhanced. The pattern may comprise straight lines. The pattern may comprise generally flat areas or circular shapes. Alternatively, the pattern may be a three-dimensional pattern, comprising for example pillars or any repeated structures.

The pattern may preferably be delimited by a series of pillars comprising InN, arranged along at least one line.

The device may preferably comprise at least one electrode for stimulating and/or monitoring contacted cells/tissues.

According to another aspect of the invention, a method for selectivel promoting the adhesion and growth of cells on a portion of surface of a substrate of an implantable device is provided. The portion is used for directly contacting biological cells or tissue. The method comprises the step of growing a layer of In₍ₓ₎Ga₍₁₋ₓ₎N on said portion, where x is in the range from 0.001 to 0.999.

Preferably, the method may comprise the steps of forming a first layer on InN on a substrate and subsequently selectively forming a second layer in In₍ₓ₎Ga₍₁₋ₓ₎N on top of said first layer said, where x is in the range from 0.001 to 0.999.

The method may further comprise the steps of forming a first layer of In₍ₓ₎Ga₍₁₋ₓ₎N, where x is in the range from 0.001 to 0.999, on a substrate, subsequently forming a second layer of InN on top of said first layer, and subsequently selectively removing portions of said second layer, to expose the first layer of In₍ₓ₎Ga₍₁₋ₓ₎N.

The second layer may preferably cover said first layer.

Preferably, said second layer of InN is selectively removed by etching.

Alternatively, said second layer of InN is selectively removed by ion beam milling.

Indium nitride, InN is shown to be a non-toxic biocompatible material which inhibits cell growth on its surface. By adding small amounts of Gallium, Ga, inside the crystal to obtain In₍ₓ₎Ga₍₁₋ₓ₎N, the inventors have shown that the resulting material can be used as a biocompatible and cell adhesive substrate. Indium-rich In₍ₓ₎Ga₍₁₋ₓ₎N enables the design of light responsive implantable substrates, which will be particularly suitable for recording and/or sending information directly from/to the cells contacted by the implant, and may also promote the controlled growth of cells by electrical or optical stimuli. In₍ₓ₎Ga₍₁₋ₓ₎N is a semiconductor material with tunable band gap. Therefore, using the invention, a plurality of field effect transistors may be provided on a substrate, wherein their sensitivity may be tuned by the amount of Ga that is added to the InN.

By patterning an InN substrate to comprise an In₍ₓ₎Ga₍₁₋ₓ₎N surface pattern, cell growth can be directed precisely along the well-defined two- or three-dimensional patterns. Specifically, the invention enables neuron growth and neuron monitoring along predefined patterns, or tailored growth of neuronal networks along predetermined paths on the substrate.

By using a substrate in accordance with the invention, tools for studying the formation and plasticity of small neural circuits can be designed in vitro. Further, as an example of in vivo applications, the substrate may be used in brain-machine interfaces, for which the synaptic connections of neurons grown on artificial substrates in accordance with the invention can be controlled and precisely directed to defined locations.

### Brief description of the drawings

Several embodiments of the present invention are illustrated by way of figures, which do not limit the scope of the invention, wherein:
- figures 1a to 1d schematically illustrate the structure of samples having a GaN, InN or In₍ₓ₎Ga₍₁₋ₓ₎N top layer respectively;
- figures 2a and 2b illustrate cell-viability results obtained using the samples of Figure 1 for the cell lines MCF10A and HS-5 respectively;
- figures 3a, 3b and 3c illustrate cell adhesion results obtained using the samples of Figure 1, wherein adhesion of different cell lines on the GaN, InN and In₍ₓ₎Ga₍₁₋ₓ₎N samples are shown in Figure 3a, 3b and 3c respectively;
- figure 4 illustrates the morphology of HEK cells grown on different substrates;
- figure 5 schematically illustrates a substrate of an implantable device according to a preferred embodiment of the invention;
- figure 6 schematically illustrates a substrate of an implantable device according to a preferred embodiment of the invention.
- figures 7a and 7b schematically illustrate methods of further In₍ₓ₎Ga₍₁₋ₓ₎N functionalization;
- figure 8a schematically illustrates a top view of a substrate of an implantable device according to a preferred embodiment of the invention;
- figures 8b and 8c schematically illustrate sides views of a substrate of an implantable device according to a preferred embodiment of the invention;
- figure 9 shows directed growth of a human neuron cell on an implantable device having a substrate according to a preferred embodiment of the invention.

### Detailed description of the invention

This section describes the invention in further detail based on preferred embodiments and on the figures. First, the materials and methods for obtaining the presented results are described. It should be understood that technical features presented for a specific embodiment may be combined with features of other embodiments, unless specifically noted otherwise.

### Materials and methods

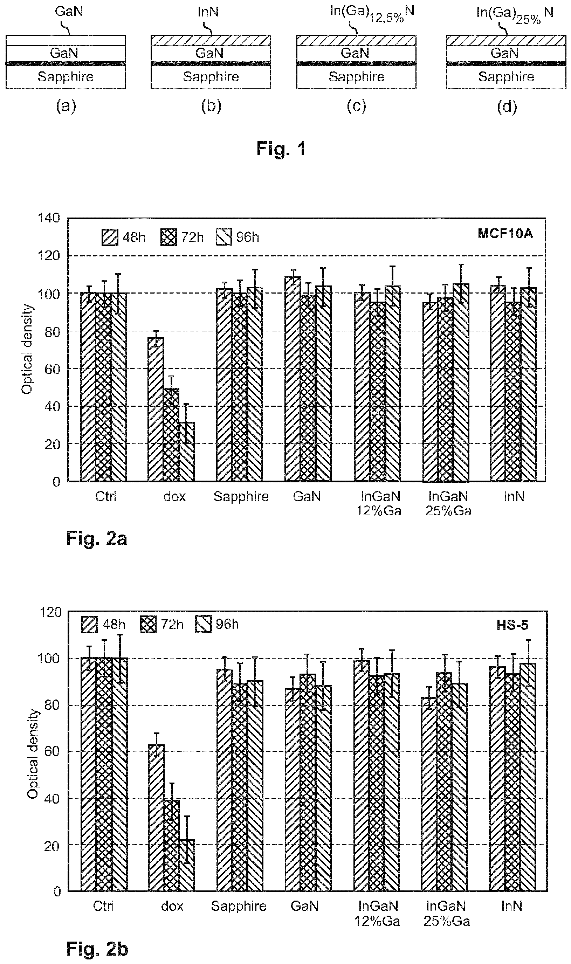

To simulate cell behavior in the presence of a device comprising a top layer, i.e. a cell-contacting layer, comprising In₍ₓ₎Ga₍₁₋ₓ₎N materials, different structures were synthesized by using Chemical Vapor Deposition on sapphire or silicon substrate. The structures are shown in Figure 1a to 1d, wherein Figure 1a shows a cell-contacting layer made of GaN, Figure 1b, shows a cell-contacting layer made of InN. Figure 1c shows a cell-contacting layer made of In₍ₓ₎Ga₍₁₋ₓ₎N, wherein 12,5 % of Ga are present. Figure 1d finally shows a cell-contacting layer made of In₍ₓ₎Ga₍₁₋ₓ₎N, wherein 25% of Ga are present. The top layer has a thickness of about 200 nm. The illustrated intermediate layers were chosen in order to optimize the transport of electrical signals from and to respective top layers. Their presence does not affect the results as to bio-adhesion and toxicity of the top layers. Cell-adhesion and -growth on Gallium nitride is known from the prior art. In order to assess the toxicity and the bio-adhesion of cells in presence of the respective top layer, Sigma XTT™ tests and bio-adhesion studies have been performed.

The experimental setup used for obtaining the reported results is described in what follows.

In order to perform adhesion microscopy, elements were placed in 24-well plates with 25 mm² element per well. 500 µl of GFP(green fluorescent protein)-PC12 suspension containing 5'000 and 10'000 cells respectively in Dulbecco's Modified Eagle's Medium, DMEM -1% horse serum with nerve growth factor 100ng/ml was added and incubated for 72 hours.

Before imaging on a Westburg Evos™ microscope, supernatants were aspirated and replaced with fresh DMEM medium without phenol red. Imaging of the elements was performed either by confocal microscopy experiments using a Zeiss LSM 510™ in fresh DMEM medium without phenol red, or by optical microscopy in reflective mode using a Zeiss™ microscope in phosphate-buffered saline, PBS.

Pfizer Adriblastina™ was used as Doxorubicin, Dox, ready-to-use injectable solution. Invitrogen™ NGF2.5S nerve growth factor was diluted in PBS/0.1% bovine serumalbumin and stored at -20°C.

Three cell lines were used in tests. First, the human mammary non-tumorigenic epithelial cell line MCF10A was grown in DMEM/F12 medium supplemented with 5% horse serum, epidermal growth factor EGF (Sigma-Aldrich™ 20ng/ml), hydrocortisone (Sigma-Aldrich™ 0.5 mg/ml), cholera toxin (Gentaur™ 100ng/ml), insulin (Sigma-Aldrich™ 10mg/ml), 100U/ml of penicillin and streptomycin.

Second, the HS-5 stromal cell line was cultivated in DMEM, 10% fetal bovine serum, 100U/ml of penicillin and streptomycin.

Third, the PC-12 cell line derived from a transplantable rat neuroendocrine tumor of the adrenal medulla was grown in 10% horse serum, 5% fetal bovine serum, 100U/ml of penicillin and streptomycin. Neuronal phenotype on this model is induced using nerve growth factor 100 ng/ml in DMEM -1% horse serum for 72 hours. PC12 cells stop dividing and terminally differentiate when treated with nerve growth factor. This makes PC12 cells useful as a model system for neuronal differentiation.

All cell lines were maintained at a temperature of 37°C in an ambient environment containing 5% CO₂ and 95% humidity.

Cell viability was determined using a colorimetric Sigma-Aldrich XTT™ assay in accordance with the manufacturer's instructions. 3'000 cells per well were plated on a 48-well plate with or without the different elements for 46 to 96 hours. Four hours before the end of the exposure, XTT was added. At the end of the exposure, optical densities of supernatants were read at 490 nm. A positive control of toxicity was obtained with doxorubicin treatment.

Figures 2a and 2b illustrate the viability of MCF10 mammary epithelial cells (Figure 2a) and HS5 stromal cells (Figure 2b) on respective cell-contacting layer of Sapphire, GaN, In₍ₓ₎Ga₍₁₋ₓ₎N (with 12,5% Ga and 25% Ga respectively) and InN. Cells were incubated for 48, 72 or 96 hours in presence of the different samples, including a control and dox sample. The latter is a positive control which induces cell death. Results are expressed in percentage (Optic Density, OD, calculated for a given sample / OD calculated for the control sample). As can be observed, the HS5 of MCF10 cell viability is not affected in the presence of In₍ₓ₎Ga₍₁₋ₓ₎N. Interestingly, the same is true for InN and Sapphire. The positive Dox control proves that the tested cells are not over-resistant.

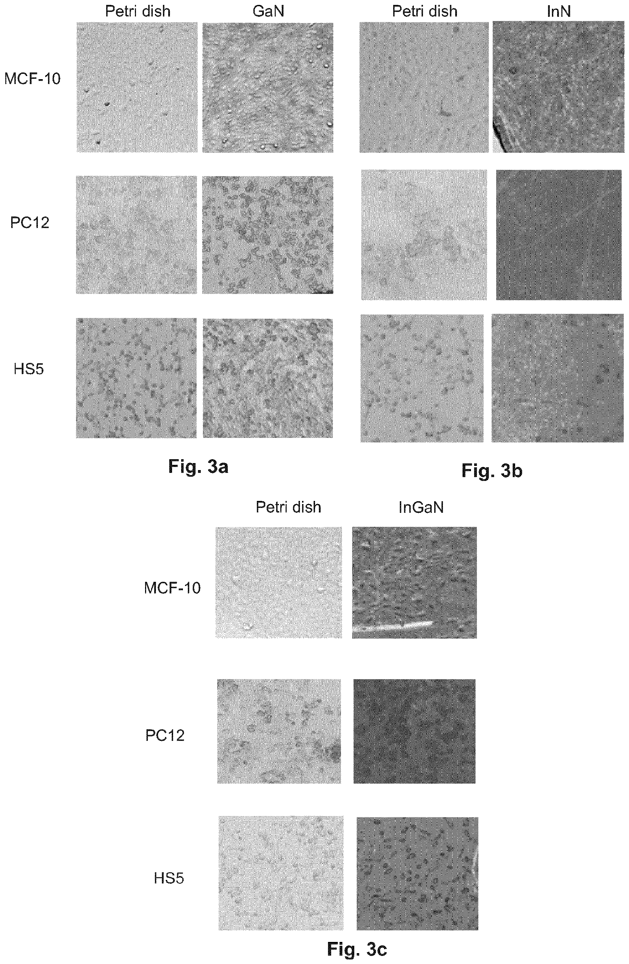

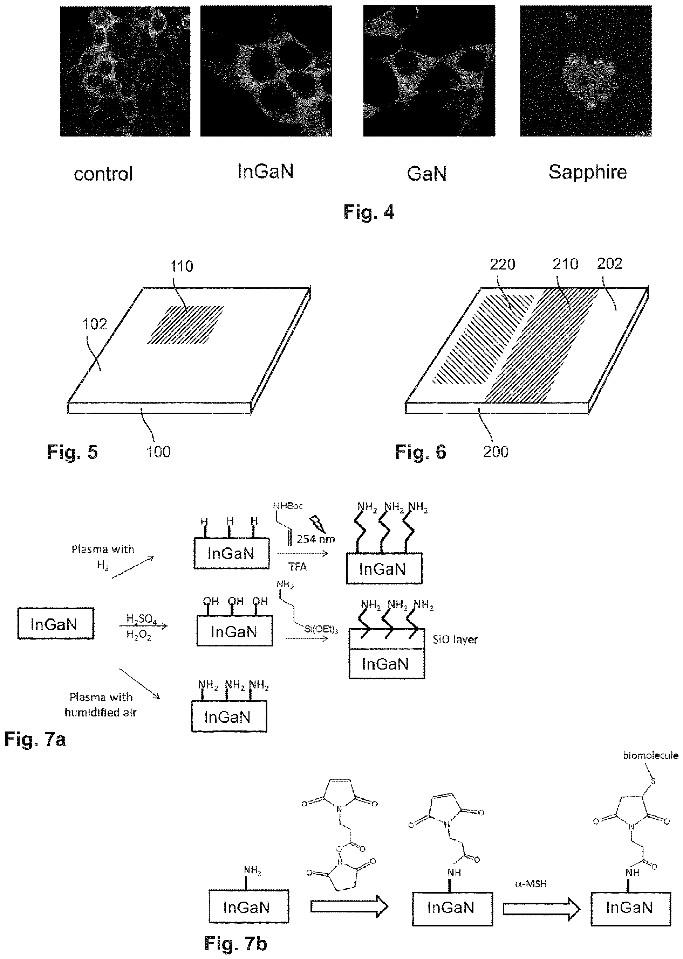

Figures 3a, 3b and 3c illustrate the cell bioadhesion results obtained as described on the different GaN, InN and In₍ₓ₎Ga₍₁₋ₓ₎N layers. Besides HS5 and MCF10 cells, PC12 cells were tested as a model for neuronal cells. In the case of InN, substantially no cells are able to adhere on the substrate. In the petri dish, cells have grown on the plastic part as in the control sample, with no sign of induced toxicity. This confirms the viability results shown in Figures 2a and 2b. It has been observed that on In₍ₓ₎Ga₍₁₋ₓ₎N, the cell growth is similar to GaN, which is surprising due to the weak proportion of Ga in the InN alloy. For HS5 cells, the cell-growth has been observed to be clearly dependent on the proportion of Ga present in the InN alloy. Therefore it is possible to control the cell growth by varying the Ga ratio in In₍ₓ₎Ga₍₁₋ₓ₎N, i.e. by varying the proportion x.
The cell shape was controlled by using Human Embryonic Kidney, HEK, cells in confocal microscopy. Figure 4 shows the morphology of the HEK cells on different samples. The HEK cells were transfected by the Green Fluorescent Protein, GFP, gene, which produces a fluorescent protein. The cell shape does not differ from the glass for GaN and In₍ₓ₎Ga₍₁₋ₓ₎N material, indicating a normal cell development on both substrates.

Neuronal cells are not able to produce collagen and can generally not stick to a glass/Saphhire substrate without precoating. Our results demonstrate the outstanding properties of InGaN which allows to promote not only adhesion but also neuronal network without any precoating.

### Methods of In₍ₓ₎Ga₍₁₋ₓ₎N Functionalization :

Several methods to functionalize GaN are described in the literature. Similar methods can be applied by the skilled person for In₍ₓ₎Ga₍₁₋ₓ₎N surfaces. This allow the In₍ₓ₎Ga₍₁₋ₓ₎N surface to be biofunctionalized / bioconjugated with biomolecules or chemical functions for specific applications such as enhancing cell selectivity or promoting cell differentiation. One of these netgids is based on photochemical "functionalized-alkene" grafting (254 nm). This reaction requires to treat GaN by H plasma in order to get Ga-H bonding. Alternatively, GaN can be oxydated by a piranha solution and then coated by "functionalized silanes" (such as APTES). However these two techniques can induce an important bandgap modification. A method has been proposed which allows to get free amino groups on the GaN surface as well as keeping electronic properties. The method uses a radio frequency glow discharge plasma with humidified air to generate amino residues. These different techniques can be used in order to generate free amino groups on In₍ₓ₎Ga₍₁₋ₓ₎N / GaN surfaces (figure 7a). Amino groups can react with hetero bifunctional linkers which contain thiol reactive moieties (such as maleimide). Biomolecules which contain thiol function or carboxylic acid derivated can be easily conjugated by this way respectively on the maleimido-GaN layer (figure 7b) or on amino layer.

### Applications:

In a preferred embodiment of the invention as illustrated in Figure 5, the substrate 100 of an implantable device comprises surface 102 having a first portion 110, which comprises In₍ₓ₎Ga₍₁₋ₓ₎N, x being in the range from 0.001 to 0.999. The presence of the In₍ₓ₎Ga₍₁₋ₓ₎N, and the proportion x selectively enhances or promotes cell adhesion to the substrate 100.

Although the substrate 100 is depicted as being planar, it may be provided in any geometrical shape. The application in which the substrate is used generally defines the constraints on its shape, which the person skilled in the art will be capable of adapting appropriately without undue burden.

The portion 110 may cover the entire surface 102 or all surfaces of the substrate. Alternatively, the substrate itself may be provided as bulk In₍ₓ₎Ga₍₁₋ₓ₎N material, and regions of different x proportions may be provided thereon to selectively tune the cell-adhesion and cell-growth rates thereon.

According to another embodiment of the invention illustrated in Figure 6, the surface 202 of the substrate 200 further comprises a second portion 220 which is distinct from said first portion 210. The second portion 220 inhibits cell-adhesion to the substrate 200, and comprises Indium nitride, InN. As both materials are non-toxic, the substrate may be used for in-vitro as well as in-vivo applications. For example, the substrate is usable for producing an implantable device which locally enhances the growth of cells, including neuronal cells.

Methods for forming thin (ranging from a thickness of a fraction of a nanometer to several micrometers) or thick layers as well as patterns of InN and/or In₍ₓ₎Ga₍₁₋ₓ₎N on a supporting substrate are as such known in the art and will not be described to any detail in the context of this invention. Specifically, a pattern of selectively cell adhesion enhancing paths or surfaces may be formed on an otherwise cell adhesion inhibiting substrate. To this end, a substrate made of InN may be locally doped with Ga along the paths or surfaces that form the pattern.

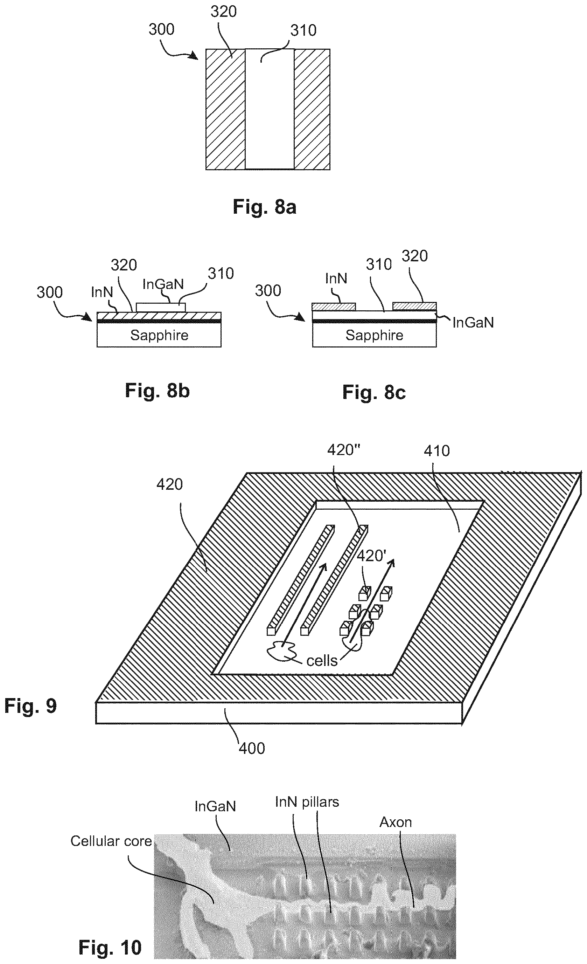

An exemplary substrate 300 is shown in a top view in Figure 8a. A first cell adhesion promoting surface portion 310 comprises In₍ₓ₎Ga₍₁₋ₓ₎N, whereas a second cell adhesion inhibiting surface portion 320 comprises InN. Such a surface pattern may be obtained using different techniques. Figure 8b is lateral view of showing a first example according to which the structure shown in Figure 8a may be obtained. A first layer of InN 320 may be provided or formed on a substrate 300. The substrate may for example be made of Saphhire. In a further step, an addition layer of In₍ₓ₎Ga₍₁₋ₓ₎N if then formed on top of the layer of InN, in location corresponding to the desired surface pattern 310. Deposition methods, including for example Metal Organic Chemical Vapor Deposition, MOCVD, and using hard-masks, to achieve the formation of such layers, are as such known in the art.

Figure 8c is a lateral view showing an alternative example according to which the structure shown in Figure 8a may be obtained. A first layer of In₍ₓ₎Ga₍₁₋ₓ₎N, is provided on the supporting substrate 300, which corresponds to the Sapphire of the previous example. The first layer may have a thickness of 4000 nm, for example. A second layer of InN is formed on top of the first layer, substantially covering the first layer integrally. The thickness of the second layer is preferably less than the thickness of the first layer, and may for example be equal to about 200 nm. The desired surface pattern 310, to which contacted cells should adhere, is then formed by locally selectively removing the the thin layer of InN 320, thereby exposing the adhesion promoting layer of In₍ₓ₎Ga₍₁₋ₓ₎N situated there below.

According to one embodiment of the invention, the removal of the top InN layer from the so-formed stack is achieved by selective etching, using lithography methods known in the art.

According to an alternative embodiment, the removal of the top InN layer is achieved by direct milling using an ion beam. This method presents the advantage that patterns presenting fine ridges and grooves of the order of 1 micrometer and having a depth/height of about 2 micrometers are achievable.

Using the above outlined techniques, several surface patterns may be formed on a substrate of an implantable device, as shown in Figure 9. The surface pattern may for example comprise surfaces of three-dimensional structures provided in/on the substrate. The pattern provided by the first portion 410 of the substrate 400, which comprises the In₍ₓ₎Ga₍₁₋ₓ₎N material and which is used to directly contact biological tissue, may for example be used to direct cell growth in a predetermined direction on the substrate of the implantable device. For example, a surface portion 410 of In₍ₓ₎Ga₍₁₋ₓ₎N may be locally delimited by pillars 420' or guiding wall structures 420" of InN, which are bio-compatible, but which do not promote cell-adhesion thereto. Cell growth of cells adhering to the first portion 410 is thereby promoting along the path delimited by the second portion 420, 420', 420", which imposes a physical limit to the cell-adhesion. The preferred direction of cell growth induced by the structures 420, 420', 420" is indicated by arrows.

Figure 10 shows a corresponding experimental result, wherein a human primary neuron cell (highlighted on the figure) is shown adhering to a first substrate portion comprising of In₍ₓ₎Ga₍₁₋ₓ₎N. An axon of the neuron cell grows along the direction which is determined by the location of pillars of InN.

The results of Figure 10 have been obtained according to the following protocol. To assess the behavior of primary neurons on patterned InGaN, GaN, or InN substrat, human primary neurons (Innoprot™, Spain) were seeded as per the manufacturer protocol at 125 000 cells/well in 48- well culture plate including patterned elements. The wells were coated with 1 ml of sterile 0.2 % gelatin solution for 1 hour and washed 3 times with sterile water solution. The different elements were washed in ethanol, rinsed with PBS and set in the wells. 1 ml of cell suspension in serum free neuronal medium supplemented by neuronal growth supplement (Innoprot™, spain) and Penicillin/Streptomycin solution was added. The medium was changed to fresh supplemented medium the next day after establishing the culture and every 3 days thereafter. After 8 days of culture, elements were washed 3 times with PBS and fixed by using 2.5% Glutaraldehyde in Sodium Cacodilate for 1h at room temperature. After 3 washes in sodium cacodilate, elements were dehydrated in ethanol (50 %, 70 % and absolute ethanol) and stored in 100 % ethanol. To exclude a potential gelatin precoating effect related to the gelatin release in culture medium, the experiment was done with subculture of neuronal cells in 96-well plate without pre-coating on unpatterned elements. 23500 cells were seeded in 200 microliters of supplemented neuronal medium for 8 days and fixed as previously described and air dried. Elements have been finally visualized by using Scanning electron microscopy.

According to a further embodiment of the invention, an implantable electronic device is provided, which comprises a substrate presenting least one surface having a portion that comprises In₍ₓ₎Ga₍₁₋ₓ₎N, which is used for directly contacting biological cells or tissue. The surface portion comprising In₍ₓ₎Ga₍₁₋ₓ₎N defines a semiconducting component of the device, the band-gap of which is a function of the proportion of Ga in the In₍ₓ₎Ga₍₁₋ₓ₎N material. The sensitivity of the semiconducting component may therefore be tuned by adjusting the proportion of Ga.
The implantable device comprises signal processing means which are as such known by the person skilled in the art and which will not be detailed in further detail in the context of the present description. The signal processing means may comprise means for amplifying or filtering a signal received from/transmitted by the semiconducting component. The signal processing means are operatively connected to the semiconducting component implemented by the In₍ₓ₎Ga₍₁₋ₓ₎N surface portion, which directly contacts the biological tissue.
The In₍ₓ₎Ga₍₁₋ₓ₎N portion of the substrate surface may therefore be used to transmit a signal emitted by the cell or tissue to the signal processing means or to transmit a processed signal to the cell or tissue. In the latter case, the implantable device may further comprise signal generating means such as a tunable voltage or current source and appropriate controlling/processing means as an input to the signal processing path. Such means are as such known to the skilled person.
In a preferred embodiment, the implantable device comprises at least one electrode, preferably arranged at an edge thereof, for contacting and selectively stimulating and/or monitoring contacted cells/tissues.
The implantable device may further comprise functionalized semiconductors such as a field effect transistors, FET, or single electrode transistors, SET, for processing or transmitting a received signal to or from the implantable device. The described components are preferably provided on a common substrate of the implantable device. In a preferred embodiment, the implantable device may be an in-vivo component of a Brain-Machine interface.
It should be understood that the detailed description of specific preferred embodiments is given by way of illustration only, since various changes and modifications within the scope of the invention will be apparent to the skilled person. The scope of protection is defined by the following set of claims.

### References

[1] Santhanam G, Ryu SI, Yu BM, Afshar A, Shenoy KV, Nature 442:195-98 (2006).
[2] Bellamkonda, R. V. Biomaterials 2006, 27 (19), 3515-3518.
[3] Turner JN, Shain W, Szarowski DH, Andersen M, Martins S, et al., Exp. Neurol. 156:33-49 (1999).
[4] Bellamkonda, R. V.; Pai, S. B.; Renaud, P. MRS Bulletin 2012, 37 (06), 557-561.
[5] Grill, W. M.; Norman, S. E.; Bellamkonda, R. V. Annu. Rev. Biomed. Eng. 2009, 11 (1), 1-24.
[6] Li, X. D.; Wang, X. N.; Bondokov, R.; Morris, J.; An, Y. H. H.; Sudarshan, T. S. Journal of Biomedical Materials Research Part B-Applied Biomaterials 2005, 72B (2), 353-361.
[7] Szarowski DH, Andersen MD, Retterer S, Spence AJ, Isaacson M, Brain Res. 983:23-35 (2003).
[8] Ito, T.; Forman, S. M.; Cao, C.; Li, F.; Eddy, C. R.; Mastro, M. A.; Holm, R. T.; Henry, R. L.; Hohn, K. L.; Edgar, J. H. Langmuir 2008, 24 (13), 6630-6635.
[9] He, W.; McConnell, G. G.; Schneider, T. G.; Bellamkonda, R. G. Adv. Mater. 2007, 19 (21), 3529-3533.
[10] Biocompatibility of semiconducting AlGaN/GaN material with living cells, Podolska, A. et al. Sensors and Actuators B 169 (2012) 401-406.

## Claims

1. An implantable device comprising a substrate (100, 200, 300, 400) having a surface (102, 202), **characterized in that**
a first portion (110, 210, 310, 410) of the surface of the substrate, which is used for directly contacting biological cells or tissue, comprises In₍ₓ₎Ga₍₁₋ₓ₎N, where x is in the range from 0.001 to 0.999, wherein the first portion promotes cell adhesion to the substrate and directs cell growth thereon, and further defines a semiconducting component of the device.

2. The device according to claim 1, wherein x is in the range from 0.01 to 0.88.

3. The device according to any of claims 1 or 2, wherein the first surface portion integrally covers the surface of the substrate.

4. The device according to any of claims 1 or 2, wherein the surface (202) further comprises a second portion (220, 320, 420) which is distinct from said first portion (210, 310, 410), wherein said second portion (220, 320, 420) inhibits cell-adhesion to the substrate, and wherein said second portion comprises Indium nitride, InN.

5. The device according to claim 4, wherein said first (210) and second (220) portions integrally cover the surface (202) of said substrate (200).

6. The device according to any of claims 1 to 5, wherein the substrate comprises Indium nitride, InN.

7. The device according to any of claims 1 to 6, wherein the first and/or second portion is provided as a thin layer on said surface of the substrate.

8. The device according to any of claims 1 to 6, wherein the first and/or second portion is provided as a thick layer on said surface of the substrate.

9. The device according to any of claims 1 to 8, wherein the first portion defines a pattern on the surface of the substrate, along which cell adhesion is enhanced.

10. The device according to claim 9, wherein said pattern comprises at least one line.

11. The device according to any of claims 9 or 10, wherein said pattern is delimited by a series of pillars comprising InN, arranged along at least one line.

12. The device according to any of claims 1 to 11, wherein the device comprises at least one electrode for stimulating and/or monitoring contacted cells/tissues.

13. An implantable device for use in a method for selectively promoting the adhesion and growth of cells on a portion of a surface of a substrate of an implantable device, wherein said portion is used for directly contacting biological cells or tissue, the method comprising the step of forming a layer of In₍ₓ₎Ga₍₁₋ₓ₎N on said portion, where x is in the range from 0.001 to 0.999.

14. The device for the use according to claim 13, wherein the method comprises the steps of forming a first layer on InN on a substrate and subsequently selectively forming a second layer in In₍ₓ₎Ga₍₁₋ₓ₎N on top of said first layer said, where x is in the range from 0.001 to 0.999.

15. The device for the use according to claim 13, wherein the method comprises the steps of forming a first layer of In₍ₓ₎Ga₍₁₋ₓ₎N, where x is in the range from 0.001 to 0.999, on a substrate, subsequently forming a second layer of InN on top of said first layer, and subsequently selectively removing portions of said second layer, to expose the first layer of In₍ₓ₎Ga₍₁₋ₓ₎N.

16. The device for the use according to claim 15, wherein said second layer covers said first layer.

17. The device for the use according to any of claims 15 or 16, wherein said second layer of InN is selectively removed by etching.

18. The device for the use according to any of claims 15 or 16, wherein said second layer of InN is selectively removed by ion beam milling.

## Patentansprüche

1. Implantierbare Vorrichtung, umfassend ein Substrat (100, 200, 300, 400), das eine Oberfläche (102, 202) aufweist, **dadurch gekennzeichnet, dass**
ein erster Abschnitt (110, 210, 310, 410) der Oberfläche des Substrats, der zum direkten Kontaktieren biologischer Zellen oder Gewebes verwendet wird, In₍ₓ₎Ga₍₁₋ₓ₎N umfasst, wobei x im Bereich von 0,001 bis 0,999 liegt, wobei der erste Abschnitt Zelladhäsion an dem Substrat fördert und Zellwachstum daran steuert, und weiter eine halbleitende Komponente der Vorrichtung definiert.

2. Vorrichtung nach Anspruch 1, wobei x im Bereich von 0,01 bis 0,88 liegt.

3. Vorrichtung nach einem der Ansprüche 1 oder 2, wobei der erste Oberflächenabschnitt die Oberfläche des Substrats integral bedeckt.

4. Vorrichtung nach einem der Ansprüche 1 oder 2, wobei die Oberfläche (202) weiter einen zweiten Abschnitt (220, 320, 420) umfasst, der von dem ersten Abschnitt (210, 310, 410) verschieden ist, wobei der zweite Abschnitt (220, 320, 420) Zelladhäsion an dem Substrat inhibiert und wobei der zweite Abschnitt Indiumnitrid, InN, umfasst.

5. Vorrichtung nach Anspruch 4, wobei der erste (210) und der zweite (220) Abschnitt die Oberfläche (202) des Substrats (200) integral bedecken.

6. Vorrichtung nach einem der Ansprüche 1 bis 5, wobei das Substrat Indiumnitrid, InN, umfasst.

7. Vorrichtung nach einem der Ansprüche 1 bis 6, wobei der erste und/oder der zweite Abschnitt als dünne Schicht auf der Oberfläche des Substrats angebracht sind.

8. Vorrichtung nach einem der Ansprüche 1 bis 6, wobei der erste und/oder der zweite Abschnitt als dicke Schicht auf der Oberfläche des Substrats angebracht sind.

9. Vorrichtung nach einem der Ansprüche 1 bis 8, wobei der erste Abschnitt ein Muster auf der Oberfläche des Substrats definiert, entlang dessen die Zelladhäsion verbessert ist.

10. Vorrichtung nach Anspruch 9, wobei das Muster mindestens eine Linie umfasst.

11. Vorrichtung nach einem der Ansprüche 9 oder 10, wobei das Muster durch eine Serie von Säulen, die InN umfassen, begrenzt ist, die entlang mindestens einer Linie angeordnet ist.

12. Vorrichtung nach einem der Ansprüche 1 bis 11, wobei die Vorrichtung mindestens eine Elektrode zum Stimulieren und/oder Überwachen kontaktierter Zellen/Gewebe umfasst.

13. Implantierbare Vorrichtung zur Anwendung in einem Verfahren zur selektiven Förderung der Adhäsion und des Wachstums von Zellen auf einem Abschnitt einer Oberfläche eines Substrats einer implantierbaren Vorrichtung, wobei der Abschnitt zum direkten Kontaktieren von biologischen Zellen oder Gewebe verwendet wird, wobei das Verfahren den Schritt des Bildens einer Schicht von In₍ₓ₎Ga₍₁₋ₓ₎N auf dem Abschnitt umfasst, wobei x im Bereich von 0,001 bis 0,999 liegt.

14. Vorrichtung zur Anwendung nach Anspruch 13, wobei das Verfahren die Schritte des Bildens einer ersten Schicht von InN auf einem Substrat und anschließend selektiv Bildens einer zweiten Schicht aus In₍ₓ₎Ga₍₁₋ₓ₎N oben auf der ersten Schicht umfasst, wobei x im Bereich von 0,001 bis 0,999 liegt.

15. Vorrichtung zur Anwendung nach Anspruch 13, wobei das Verfahren die Schritte des Bildens einer ersten Schicht von In₍ₓ₎Ga₍₁₋ₓ₎N, wobei x im Bereich von 0,001 bis 0,999 liegt, auf einem Substrat, anschließend Bildens einer zweiten Schicht von InN oben auf der ersten Schicht, und anschließend selektiv Entfernens von Abschnitten der zweiten Schicht, um die erste Schicht von In₍ₓ₎Ga₍₁₋ₓ₎N freizulegen, umfasst.

16. Vorrichtung zur Anwendung nach Anspruch 15, wobei die zweite Schicht die erste Schicht bedeckt.

17. Vorrichtung zur Anwendung nach einem der Ansprüche 15 oder 16, wobei die zweite Schicht von InN durch Ätzen selektiv entfernt wird.

18. Vorrichtung zur Anwendung nach einem der Ansprüche 15 oder 16, wobei die zweite Schicht von InN durch lonenstrahlfräsen selektiv entfernt wird.

## Revendications

1. Dispositif implantable comprenant un substrat (100, 200, 300, 400) possédant une surface (102, 202), **caractérisé en ce qu'**une première portion (110, 210, 310, 410) de la surface du substrat, qui est utilisée pour une mise en contact directe avec un tissu ou des cellules biologiques, comprend du In₍ₓ₎Ga₍₁₋ₓ₎N, où x se situe dans la plage de 0,001 à 0,999 ; dans lequel la première portion favorise l'adhérence cellulaire au substrat et dirige la croissance cellulaire sur ce dernier ; et définit en outre un composant du dispositif faisant office de semiconducteur.

2. Dispositif selon la revendication 1, dans lequel x se situe dans la plage de 0,01 à 0,88.

3. Dispositif selon l'une quelconque des revendications 1 ou 2, dans lequel la première portion de la surface recouvre de manière intégrale la surface du substrat.

4. Dispositif selon l'une quelconque des revendications 1 ou 2, dans lequel la surface (202) comprend en outre une seconde portion (220, 320, 420) qui est distincte de ladite première portion (210, 310, 410) ; dans lequel ladite seconde portion (220, 320, 420) inhibe l'adhérence cellulaire au substrat ; et dans lequel ladite seconde portion comprend du nitrure d'indium, InN.

5. Dispositif selon la revendication 4, dans lequel ladite première portion (210) et ladite seconde portion (220) recouvrent de manière intégrale la surface (202) dudit substrat (200).

6. Dispositif selon l'une quelconque des revendications 1 à 5, dans lequel le substrat comprend du nitrure d'indium, InN.

7. Dispositif selon l'une quelconque des revendications 1 à 6, dans lequel la première portion et/ou la seconde portion sont prévues sous la forme d'une couche mince sur ladite surface du substrat.

8. Dispositif selon l'une quelconque des revendications 1 à 6, dans lequel la première portion et/ou la seconde portion sont prévues sous la forme d'une couche épaisse sur ladite surface du substrat.

9. Dispositif selon l'une quelconque des revendications 1 à 8, dans lequel la première portion définit un motif sur la surface du substrat, le long duquel l'adhérence cellulaire est favorisée.

10. Dispositif selon la revendication 9, dans lequel ledit motif comprend au moins une ligne.

11. Dispositif selon l'une quelconque des revendications 9 ou 10, dans lequel ledit motif est délimité par une série de colonnes contenant du InN, arrangées le long d'au moins une ligne.

12. Dispositif selon l'une quelconque des revendications 1 à 11, dans lequel le dispositif comprend au moins une électrode pour stimuler et/ou surveiller des cellules/tissus mis en contact.

13. Dispositif implantable pour son utilisation dans un procédé destiné à favoriser de manière sélective l'adhérence et la croissance de cellules sur une portion d'une surface d'un substrat d'un dispositif implantable ; dans lequel ladite portion est utilisée pour une mise en contact directe avec un tissu ou des cellules biologiques, le procédé comprenant l'étape consistant à former une couche de In₍ₓ₎Ga₍₁₋ₓ₎N sur ladite portion, où x se situe dans la plage de 0,001 à 0,999.

14. Dispositif pour son utilisation selon la revendication 13, dans lequel le procédé comprend les étapes consistant à former une première couche de InN sur un substrat et par la suite à former de manière sélective une deuxième couche de In₍ₓ₎Ga₍₁₋ₓ₎N par-dessus ladite première couche, où x se situe dans la plage de 0,001 à 0,999.

15. Dispositif pour son utilisation selon la revendication 13, dans lequel le procédé comprend les étapes consistant à former une première couche de In₍ₓ₎Ga₍₁₋ₓ₎N, où x se situe dans la plage de 0,001 à 0,999, sur un substrat, à former par la suite une deuxième couche de InN par-dessus ladite première couche, et à ensuite retirer de manière sélective des portions de ladite deuxième couche, dans le but d'exposer la première couche de In₍ₓ₎Ga₍₁₋ₓ₎N.

16. Dispositif pour son utilisation selon la revendication 15, dans lequel ladite deuxième couche recouvre ladite première couche.

17. Dispositif pour son utilisation selon l'une quelconque des revendications 15 ou 16, dans lequel ladite deuxième couche de InN est éliminée de manière sélective par gravure.

18. Dispositif pour son utilisation selon l'une quelconque des revendications 15 ou 16, dans lequel ladite deuxième couche de InN est éliminée de manière sélective via un usinage par faisceau ionique.
